# EUROPEAN PATENT SPECIFICATION

(11) **EP 4 360 763 B1**
(45) Date of publication and mention of the grant of the patent: **17.12.2025**
(21) Application number: 23765133.6
(22) Date of filing: 01.06.2023
(51) Int. Cl.: A61M 15/06, A61M 11/02, B02C 25/00, B02C 18/14, B02C 18/18, B02C 18/22, B02C 18/24, A61M 11/00, A61M 15/00

(54) **INHALER**
INHALATOR
INHALATEUR

(30) Priority: 14.09.2022 KR 20220115383
(43) Date of publication of application: 01.05.2024
(73) Proprietor: KT&G Corporation, Daedeok-gu Daejeon 34337 (KR)
(72) Inventor: KIM, Tae Heon, Daejeon 34128 (KR); KIM, Jae Hyun, Daejeon 34128 (KR); LEE, Mi Jeong, Daejeon 34128 (KR); JEONG, Minseok, Daejeon 34128 (KR); JUNG, Yongmi, Daejeon 34128 (KR); JEOUNG, Eunmi, Daejeon 34128 (KR); CHUNG, Tae Young, Daejeon 34128 (KR); HAN, Seung Kyu, Daejeon 34128 (KR)
(74) Representative: Ter Meer Steinmeister & Partner
(86) International application number: PCT/KR2023/007558
(87) International publication number: WO 2024/058343

(56) References cited:
- WO-A1-2023/229175
- JP-A- 2009 131 367
- JP-A- H09 322 938
- KR-B1- 100 813 784
- US-A- 4 576 157
- US-A1- 2001 013 342

## Description

### Technical Field

Disclosed is an inhaler.

### Background Art

In general, an inhaler is a device used to inhale a composition such as medication through the oral cavity or nasal cavity as a liquid or gas in the process of inhalation. Such an inhaler may include a container accommodating an inhalable composition, and the composition may be sprayed from the container through a thin tube to the oral cavity or nasal cavity through an intake to be inhaled by a user. US4576157A1 discloses an inhaler with a nozzle and a needle valve.

The above description has been possessed or acquired by the inventor(s) in the course of conceiving the present disclosure and is not necessarily an art publicly known before the present application is filed. WO 2023/229175 A1 relates to an inhaler which may comprise: a housing having one surface, another surface opposite to the one surface, and a plurality of side surfaces connecting the one surface and the other surface; a mouthpiece disposed on the one surface of the housing; a reservoir disposed in the housing and storing an inhalable composition; a nozzle extending from the mouthpiece to the reservoir; and a needle valve movably disposed in the nozzle, wherein when a suction force is not applied to the mouthpiece, the needle valve is maintained in a first state in which the nozzle is closed, and when a suction force is applied through the mouthpiece, the needle valve is switched to a second state in which the nozzle is opened.

### Prior Document: Korean Patent Gazette No. 10-1759972

### Disclosure of the Invention

### Technical Goals

An object according to an embodiment is to provide an inhaler having a technology in which a flow path for ejecting an aerosol is divided into two spaces having different cross-sectional areas according to an operation of a valve and an appropriate section of the two divided spaces is specified and aerosol formation conditions are derived to enable uniform ejection of a sprayed aerosol.

The technical tasks obtainable from the present disclosure are non-limited by the above-mentioned technical tasks. And, other unmentioned technical tasks can be clearly understood from the following description by those having ordinary skill in the technical field to which the present disclosure pertains.

### Technical Solutions

The invention is defined by the appended claims.

An inhaler according to an embodiment for achieving the above objects includes: a housing having one surface, the other surface opposite to the one surface, and a plurality of side surfaces connecting the one surface and the other surface; a mouthpiece which is disposed on the one surface of the housing; a reservoir which is disposed inside the housing and stores an inhalable composition; a nozzle which extends from the mouthpiece to the reservoir; a needle valve which is movably disposed inside the nozzle and operates in a first state for closing the nozzle and a second state for opening the nozzle; and a sealing member which is fixedly provided on the nozzle. In the first state, a first gap is formed between the needle valve and the nozzle, and in the second state, a second gap is formed between the needle valve and the sealing member.

According to an aspect, the first state may be defined as a state in which the needle valve comes into contact with the sealing member, and the second state may be defined as a state in which the needle valve does not come into contact with the sealing member.

According to an aspect, the first gap may be smaller than the second gap.

According to an aspect, the nozzle may include a first nozzle portion adjacent to the mouthpiece; and a second nozzle portion adjacent to the reservoir. The first nozzle portion may be formed to have a diameter smaller than a diameter of the second nozzle portion.

According to an aspect, the needle valve may include: a first valve portion which is formed on a front end and vertically reciprocates in a first direction from the other surface to the one surface of the housing or a second direction from the one surface to the other surface of the housing through the first nozzle portion and the second nozzle portion; and a second valve portion which is formed on a lower portion of the first valve portion. The first valve portion may be formed to have a diameter smaller than a diameter of the second valve portion.

According to an aspect, the first valve portion may be formed to have the diameter smaller than the diameter of the first nozzle portion.

According to an aspect, the first valve portion may not come into contact with the first nozzle portion in the first state or the second state, and the second valve portion may come into contact with the sealing member in the first state and may not come into contact with the sealing member in the second state.

According to an aspect, the first gap may be formed between the first valve portion and the first nozzle portion, and the second gap may be formed between the first valve portion and the sealing member.

According to an aspect, the second gap may allow movement of the inhalable composition discharged from the reservoir, and the first gap may allow the movement of the inhalable composition to the mouthpiece by controlling a particle size of the inhalable composition passing through the second gap.

According to an aspect, the first gap may be formed to 0.015 millimeters (mm) to 0.03 mm.

According to an aspect, the inhaler may further include: a piston which has one surface coupled to the needle valve, and vertically reciprocates in a first direction from the other surface to the one surface of the housing or a second direction from the one surface to the other surface of the housing; and a spring which is provided below the piston in a preloaded state. When a suction force is not applied through the mouthpiece, the spring may push the piston up in the first direction to maintain the first state, and when a suction force is applied through the mouthpiece, the piston may overcome preload of the spring and move in the second direction to switch the state into the second state.

According to an aspect, the inhaler may further include a passage which is formed inside the housing and extends from one side of the mouthpiece to a bottom portion of the piston. The passage may transfer the suction force applied to the mouthpiece to the piston.

According to an aspect, the inhaler may further include a negative pressure forming portion which forms a space between the piston and the passage. The negative pressure forming portion may induce the piston to move in the second direction by generating a negative pressure by the suction force.

### Effects

According to the inhaler according to an embodiment, there are effects that a flow path for ejecting an aerosol is divided into two spaces having different cross-sectional areas according to an operation of a valve and an appropriate section of the two divided spaces is specified and aerosol formation conditions are derived to enable uniform ejection of a sprayed aerosol.

The effects of the inhaler are not limited to the above-mentioned effects, and other unmentioned effects can be clearly understood from the following description by one of ordinary skill in the art to which the present disclosure pertains.

### Brief Description of Drawings

FIG. 1 is a perspective view of an inhaler according to an embodiment.
FIG. 2 is a cross-sectional view of an inhaler according to an embodiment.
FIG. 3 is a cross-sectional view of an inhaler according to an embodiment.
FIG. 4 illustrates an inhaler in a first state and a second state.

The accompanying drawings illustrate desired embodiments of the present disclosure and are provided together with the detailed description for better understanding of the technical idea of the present disclosure. Therefore, the present disclosure should not be construed as being limited to the embodiments set forth in the drawings.

### Best Mode for Carrying Out the Invention

Hereinafter, embodiments will be described in detail with reference to the illustrative drawings. Regarding the reference numerals assigned to the components in the drawings, it should be noted that the same components will be designated by the same reference numerals, wherever possible, even though they are shown in different drawings. Further, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe constituent elements of the embodiments. These terms are used only for the purpose of discriminating one constituent element from another constituent element, and the nature, the sequences, or the orders of the constituent elements are not limited by the terms. When one constituent element is described as being "connected", "coupled", or "attached" to another constituent element, it should be understood that one constituent element can be connected or attached directly to another constituent element, and an intervening constituent element can also be "connected", "coupled", or "attached" to the constituent elements.

The same name may be used to describe an element included in the embodiments described above and an element having a common function. Unless otherwise mentioned, the descriptions on the embodiments may be applicable to the following embodiments and thus, duplicated descriptions will be omitted for conciseness.

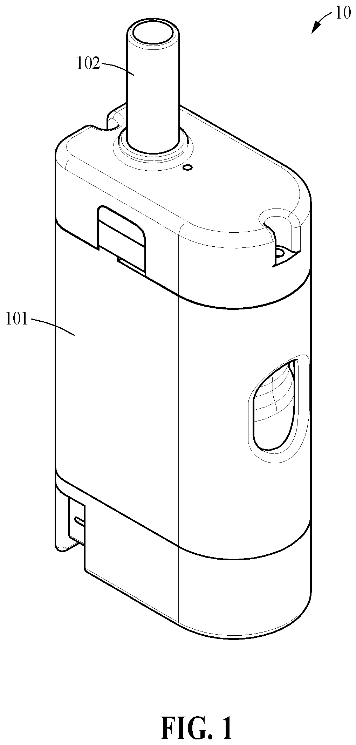

FIG. 1 is a perspective view of an inhaler 10 according to an embodiment.

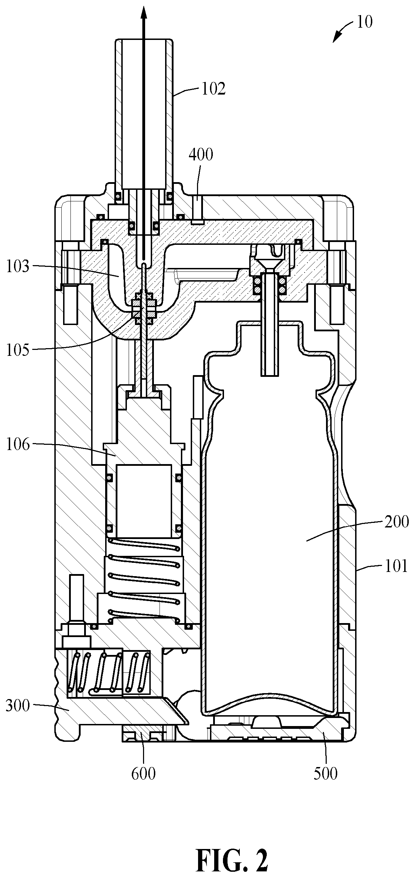

FIG. 2 is a cross-sectional view of the inhaler 10 according to an embodiment.

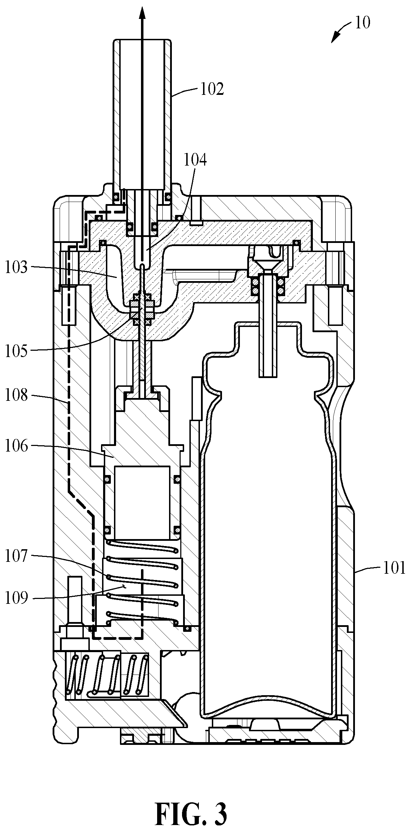

FIG. 3 is a cross-sectional view of the inhaler 10 according to an embodiment.

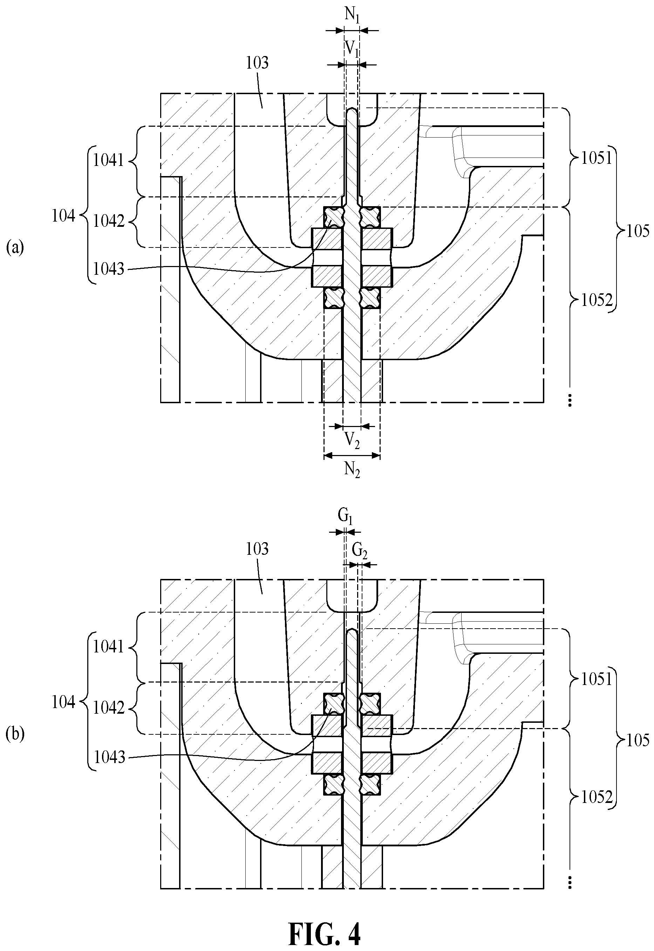

FIG. 4 illustrates the inhaler 10 in a first state and a second state.

Referring to FIG. 1, the inhaler 10 according to an embodiment may include a housing 101 and a mouthpiece 102.

The housing 101 may include a first surface formed on one surface, a second surface opposite to the first surface, and a plurality of side surfaces connecting the first surface and the second surface. The first surface of the housing 101 may be, for example, a surface located on the top of the housing 101, and the second surface may be, for example, a bottom surface of the housing 101. Hereinafter, a direction from the second surface to the first surface is defined as a first direction, and a direction from the first surface to the second surface is defined as a second direction.

The mouthpiece 102 may be disposed on the first surface of the housing 101. A user may inhale an inhalable composition accommodated in the inhaler 10 through the mouthpiece 102. At this time, the user may inhale the composition, for example, in the form of an aerosol or in the form of a powder. Hereinafter, the inhaler 10 according to an embodiment will be described using the inhaler 10 that sprays an inhalable composition in the form of an aerosol as an example.

Referring to FIG. 2, in the inhaler 10 according to an embodiment, a canister 200 accommodating an inhalable composition may be mounted on the inside of the housing 101, and a certain amount of the composition of the canister 200 may be filled in a reservoir 103. The user may visually identify a remaining amount of the composition stored in the reservoir 103 through a viewing window (not shown) provided on the housing 101. A filling lever 300 that may be pressed by the user may be provided to fill the inside of the reservoir 103 with the composition accommodated in the canister 200. The filling lever 300 may be positioned on the second surface of the housing 101, and when the user presses the filling lever 300, the filling lever 300 may push up a bottom surface of the canister 200 in the first direction so that an injection hole of the canister 200 communicates with the reservoir 103, and the composition may move from the canister 200 to the reservoir 103 through the injection hole. In addition, the inhaler 10 according to an embodiment may include the counter (not shown) that counts the number of times of filling in connection with a vertical movement of the canister 200 during the filling of the reservoir 103, and a display window (not shown) that displays a remaining amount of the composition in the canister 200. The composition stored in the reservoir 103 may be sprayed in the form of an aerosol as the user applies a suction force to the mouthpiece 102. At this time, an inhalation interlocking valve 105 that opens and closes the reservoir 103 by the suction force may operate, and the opening and closing operation of the inhalation interlocking valve 105 may be controlled by a piston 106. Meanwhile, a relief valve (not shown) may be provided on the reservoir 103, and a relief vent hole 400 may be provided on the first surface of the housing 101. The relief valve is interlocked with the filling lever 300 through a relief bar (not shown) or a relief bar movement protrusion (not shown), and accordingly, the relief valve may be opened first to discharge a residual gas in the reservoir 103, before the filling lever 300 pushes the canister 200 up in the first direction to fill the reservoir 103. The inhaler 10 according to an embodiment may further include a cover 500 and a locking device 600 for preventing the canister 200 from being separated.

In the inhaler 10 described above, a needle valve method developed to solve problems that occur when an aerosol is sprayed into the user's nasal cavity or oral cavity is applied to the inside of the housing 101, and this needle valve method will be described in detail below with reference to FIGS. 3 and 4.

In general, when a pinch valve method is applied to an inhaler, a problem of wetting the inside of the user's oral cavity may occur due to too large particles of the aerosol sprayed through a nozzle having a circular cross-sectional area. In addition, since a tip of an injection nozzle is positioned inside the oral cavity, a problem in that the aerosol hits the oral cavity hard during the spraying may occur. In order to solve such problems, the needle valve method is applied in the present disclosure.

Referring to FIG. 3, the inhaler 10 according to an embodiment may further include the reservoir 103, a nozzle 104, and a needle valve 105.

The reservoir 103 may be disposed inside the housing 101. The reservoir 103 may store an inhalable composition.

The nozzle 104 may allow the mouthpiece 102 to communicate with the reservoir 103. The nozzle 104 may be provided as a tube extending from the mouthpiece 102 to the reservoir 103.

The needle valve 105 may be movably disposed inside the nozzle 104. For example, the needle valve 105 may move up and down inside the nozzle 104. The needle valve 105 may open or close the nozzle 104 depending on its position within the nozzle 104. That is, the needle valve 105 may open the nozzle 104 so that the mouthpiece 102 and the reservoir 103 communicate with each other, or may close the nozzle 104 so that the mouthpiece 102 and the reservoir 103 are isolated from each other.

In the needle valve method using the needle valve 105, as the needle-shaped valve 105 is positioned in the middle of the nozzle 104, a cross-sectional area for spraying an aerosol may maintain a donut shape. In addition, finer particles may be formed by spraying the aerosol from a narrower section compared to the same injection area, and a phenomenon of hitting or wetting the inside of the oral cavity may be improved, because the nozzle 104 for spraying an aerosol is positioned at a lower tip of the mouthpiece 102 which is far from the oral cavity.

The nozzle 104 may be switched to one of a first state or a second state by the needle valve 105. The first state is a state in which the nozzle 104 is closed, and the second state is a state in which the nozzle 104 is open. In the first state, the mouthpiece 102 and the reservoir 103 may be isolated from each other. In the second state, the mouthpiece 102 and the reservoir 103 may communicate with each other. That is, in the second state, the inhalable composition stored in the reservoir 103 may be discharged to the mouthpiece 102 through the nozzle 104 as indicated by an arrow.

In addition, the needle valve 105 may operate in association with suction.

Specifically, when a suction force is not applied to the mouthpiece 102, the needle valve 105 may maintain the nozzle 104 in the first state. When a suction force is applied through the mouthpiece 102, the needle valve 105 may move in the second direction to switch the nozzle 104 into the second state.

Referring back to FIG. 3, the inhaler 10 according to an embodiment may further include the piston 106, a spring 107, a passage 108, and a negative pressure forming portion 109.

The piston 106 may be disposed inside the housing 101, and one end of the needle valve 105 may be coupled to one surface of the piston 106. The piston 106 may reciprocate vertically in a cylinder.

The spring 107 may be provided below the piston 106. At this time, the spring 107 may be provided in a preloaded state.

The passage 108 may be formed inside the housing 101. The passage 108 may allow the mouthpiece 102 to communicate with the piston 106. Specifically, one end of the passage 108 may be connected to one side of the mouthpiece 102, and the other end of the passage 108 may be connected to the bottom of the piston 106. At this time, one end of the passage 108 may be connected to the mouthpiece 102 at a position spaced apart from a position where the nozzle 104 is connected to the mouthpiece 102. The passage 108 may transfer a suction force applied to the mouthpiece 102 to the piston 106.

The negative pressure forming portion 109 may be formed between the piston 106 and one end of the passage 108 connected to the piston 106. The negative pressure forming portion 109 may form a space between the piston 106 and the passage 108. When a suction force is applied to the mouthpiece 102, the suction force transferred through the passage 108 may reach the negative pressure forming portion 109, and the negative pressure forming portion 109 may generate a negative pressure. Accordingly, the negative pressure forming portion 109 may induce the piston 106 to move in the second direction.

As a result, when a suction force is not applied to the mouthpiece 102, the spring 107 may push the piston 106 up in the first direction so that the needle valve 105 and the nozzle 104 are maintained in the first state. On the other hand, when a suction force is applied to the mouthpiece 102, the suction force may be transferred to the negative pressure forming portion 109 through the passage 108, and the piston 106 may overcome the preload of the spring 107 by the negative pressure generated by the negative pressure forming portion 109, and move in the second direction. Accordingly, the needle valve 105 may move in the second direction and the nozzle 104 may be switched into the second state.

Hereinafter, referring to FIG. 4, the nozzle 104 and the needle valve 105 in the first state and the second state will be described in more detail, and the configuration in which two gaps G1 and G2 having different cross-sectional areas are formed on the nozzle 104 according to the operation of the needle valve 105 to control a spraying amount of an aerosol will be described in detail.

FIG. 4A shows the inhaler 10 in the first state and FIG. 4B shows the inhaler 10 in the second state.

In the first state, the nozzle 104 and the needle valve 105 may come into contact with each other and the mouthpiece 102 and the reservoir 103 may be isolated from each other.

In the second state, the needle valve 105 may move in the second direction so that the nozzle 104 and the needle valve 105 may not come into contact with each other, and the mouthpiece 102 and the reservoir 103 may communicate with each other through the nozzle 104. Accordingly, the inhalable composition in the reservoir 103 may be discharged outside from the reservoir 103 through the mouthpiece 102.

Specifically, the nozzle 104 may include a first nozzle portion 1041 and a second nozzle portion 1042.

The first nozzle portion 1041 may be a portion adjacent to the mouthpiece 102.

The second nozzle portion 1042 may be, for example, a portion located on the bottom of the first nozzle portion 1041 and adjacent to the reservoir 103.

The nozzle 104 may be formed such that the first nozzle portion 1041 has a diameter smaller than that of the second nozzle portion 1042.

The needle valve 105 may include a first valve portion 1051 and a second valve portion 1052.

The first valve portion 1051 may be a portion formed at a front end of the needle valve 105. The first valve portion 1051 may move adjacent to the first nozzle portion 1041 or the second nozzle portion 1042 when the piston 106 moves vertically. For example, in the first state, the first valve portion 1051 may be disposed adjacent to the first nozzle portion 1041. In addition, in the second state, the first valve portion 1051 may move in the second direction to be adjacent to the second nozzle portion 1042.

The second valve portion 1052 may be, for example, a portion formed below the first valve portion 1051. A lower end of the second valve portion 1052 may be coupled to one surface of the piston 106. Accordingly, when the piston 106 vertically reciprocates, the needle valve 105 may vertically reciprocate.

The needle valve 105 may be formed such that the first valve portion 1051 has a diameter smaller than that of the second valve portion 1052.

In addition, the first valve portion 1051 may be formed to have a diameter smaller than that of the first nozzle portion 1041. That is, the first valve portion 1051 may not come into contact with any of the first nozzle portion 1041 and the second nozzle portion 1042 in the first state or the second state.

Meanwhile, the nozzle 104 may further include a sealing member 1043 provided on the second nozzle portion 1042. The sealing member 1043 is provided as a Quad-ring formed of an elastic material such as silicon or rubber. An inner diameter of the sealing member 1043 may be larger than the diameter of the first valve portion 1051 and may be equal to or smaller than the diameter of the second valve portion 1052. Also, the inner diameter of the sealing member 1043 may be larger than the diameter of the first nozzle portion 1041.

The second valve portion 1052 may be disposed adjacent to the sealing member 1043 in the first state. That is, in the first state, the second valve portion 1052 may come into contact with the sealing member 1043. Accordingly, the reservoir 103 may be sealed airtightly so that no aerosol leaks through the nozzle 104.

On the other hand, the second valve portion 1052 may move in the second direction with respect to the sealing member 1043 and may not to come into contact with the nozzle 104 in the second state. Accordingly, the inhalable composition stored in the reservoir 103 may be discharged to the mouthpiece 102 through the nozzle 104.

Referring to FIG. 4B, a gap formed between the first nozzle portion 1041 and the first valve portion 1051 is defined as a first gap G1, and a gap formed between the sealing member 1043 and the first valve portion 1051 is defined as a second gap G2. Due to the structure of the nozzle 104 and the needle valve 105 described above, the first gap G1 may be formed to be smaller than the second gap G2.

The reason for forming the diameter of the needle valve 105 differently according to the section and spatially dividing the nozzle 104 so that the first gap G1 and the second gap G2 having different cross-sectional areas are formed as described above is to uniformly control the spraying amount of the aerosol.

The spraying amount of the aerosol is generally proportional to the cross-sectional area of the nozzle 104, and if the nozzle 104 is not spatially divided into the first gap G1 and the second gap G2, the cross-sectional area of the nozzle 104 may be determined only by a gap between the sealing member 1043 and the needle valve 105. According to the invention, the sealing member 1043 is provided as a Quad-ring having a tolerance of 0.05 mm, and thus, it is difficult to ensure a cross-sectional area for uniform spraying. Accordingly, the nozzle 104 may be spatially divided so that only the opening and closing of the inhalable composition is controlled in the second gap G2, and the aerosol is finally sprayed in the first gap G1 where the tolerance is more easily controlled.

When the opening and spraying occur simultaneously, the spraying amount of the aerosol may be controlled only by a spraying area of the first gap G1.

Factors affecting the spraying amount may include pipe resistance, propulsion pressure, fluid viscosity, and the like, as well as the cross-sectional area of the nozzle described above.

Specifically, the spraying amount of the aerosol may vary depending on a distance that the inhalable composition moves to the first gap G1. When the second gap G2 is the same, the spraying amount of the aerosol may vary depending on a stroke distance of the needle valve 105. For example, when the stroke distance of the needle valve 105 is shortened, the distance through which the inhalable composition passes through the first gap G1 may increase and the spraying amount may decrease.

The first gap G1 may be formed in the first state or the second state. The second gap G2 is formed when the first valve portion 1051 is disposed adjacent to the sealing member 1043, and therefore, the second gap G2 may be formed in the second state in which the needle valve 105 is moved in the second direction.

The second gap G2 may allow the movement of the inhalable composition discharged from the reservoir 103. The first gap G1 may control a particle size of an aerosol which has passed through the second gap G2. That is, since the first gap G1 is smaller than the second gap G2, the first gap G1 may allow only the movement of an aerosol with fine particles from the aerosol. As a result, only an aerosol having a size that is able to pass through the first gap G1 may move to the mouthpiece 102 to be discharged to the user.

That is, when the nozzle 104 is opened by a suction force, the aerosol is finally sprayed to the outside through the first gap G1, but the initial discharge from the reservoir 103 may be performed through the second gap G2 formed between the sealing member 1043 and the first valve portion 1051.

That is, since both the nozzle 104 and the needle valve 105 are formed straight, the first gap G1 is constantly formed regardless of the opening and closing of the nozzle 104, and therefore, the opening and closing is substantially performed between the sealing member 1043 and the needle valve 105.

In this case, the first gap G1 may be formed to have a size of 0.015 millimeters (mm) to 0.03 mm for the spraying of an aerosol with sufficiently small particles. For example, when the first gap G1 is smaller than 0.015 mm, it is difficult for a liquid composition to move along a narrow gap, and thus, a gas may be mainly sprayed, and some liquid droplets may be weakly sprayed as if it is boiling. On the other hand, when the first gap G1 is larger than 0.03 mm, large liquid droplets are mainly sprayed, which may cause an extremely large spraying amount per unit time, and the user may feel that the nasal cavity or oral cavity is wet. In addition, when the second gap G2 is formed too narrow due to a manufacturing tolerance of the sealing member 1043, a phenomenon which is the same phenomenon occurring due to the narrow first gap G1 may occur, and therefore, in consideration of this point, the second gap G2 may be formed sufficiently larger than the first gap G1. Accordingly, the inhaler 10 according to an embodiment may spray an aerosol with fine particles and easily adjust the spraying amount.

As described above, in the inhaler 10 according to an embodiment, the first gap G1 and the second gap G2 having different cross-sectional areas may be divided on the nozzle 104 for ejecting the aerosol according to the opening and closing operation of the needle valve 105, and an appropriate section of the two divided gaps G1 and G2 is specified to enable uniform ejection of a sprayed aerosol.

In addition, as the aerosol is sprayed in a narrower section compared to the injection area by the first gap G1 and the second gap G2 having the donut shape, finer particles may be formed and a phenomenon of wetting or hitting the inside of the user's mouth may be improved.

In addition, the inhaler 10 according to an embodiment may maintain a state in which the nozzle 104 is closed by receiving a force of the needle valve 105 normally pushing up in the first direction due to the preload of the spring 107. However, in the inhaler 10 according to an embodiment, when a suction force is applied through the mouthpiece 102 by the user, a negative pressure may be formed on the bottom of the piston 106, the piston 106 may move downward by overcoming the preload of the spring 107, and the needle valve 105 connected to the piston 106 may move downward, thereby forming the second gap G2 between the sealing member 1043 and the needle valve 105. Accordingly, the inhalable composition in the reservoir 103 may be discharged to the outside, for example, to the user's oral cavity through the first gap G1 by passing through the second gap G2 in the form of an aerosol. When the user stops inhaling, the piston 106 and the needle valve 105 may move upward again due to a restoring force of the spring 107 and the nozzle 104 may be closed to stop the spraying of the aerosol.

While the embodiments of the present disclosure have been described above with reference to specific components, and limited embodiments and drawings, the above descriptions are merely for better understanding of the present disclosure Accordingly, the scope of the present disclosure is defined not by the detailed description, but by the appended claims.

## Claims

1. An inhaler (10) comprising:
a housing (101) having one surface, the other surface opposite to the one surface, and a plurality of side surfaces connecting the one surface and the other surface;
a mouthpiece (102) which is disposed on the one surface of the housing (101);
a reservoir (103) which is disposed inside the housing (101) and stores an inhalable composition;
a nozzle (104) which extends from the mouthpiece (102) to the reservoir (103);
a needle valve (105) which is movably disposed inside the nozzle (104) and operates in a first state for closing the nozzle (104) and a second state for opening the nozzle (104); and
a sealing member (1043) which is fixedly provided on the nozzle (104),
wherein, in the first state, a first gap (G1) is formed between the needle valve (105) and the nozzle (104),
wherein, in the second state, a second gap (G2) is formed between the needle valve (105) and the sealing member (1043),
wherein the sealing member (1043) is provided as a Quad-ring formed of an elastic material, and
wherein a tolerance of the Quad-ring is 0.05 mm.

2. The inhaler (10) of claim 1,
wherein the first state is defined as a state in which the needle valve (105) comes into contact with the sealing member (1043), and
wherein the second state is defined as a state in which the needle valve (105) does not come into contact with the sealing member (1043).

3. The inhaler (10) of claim 1, wherein the first gap (G1) is smaller than the second gap (G2).

4. The inhaler (10) of claim 1,
wherein the nozzle (104) comprises:
a first nozzle portion (1041) adjacent to the mouthpiece (102); and
a second nozzle portion (1042) adjacent to the reservoir (103), and
wherein the first nozzle portion (1041) is formed to have a diameter smaller than a diameter of the second nozzle portion (1042).

5. The inhaler (10) of claim 4,
wherein the needle valve (105) comprises:
a first valve portion (1051) which is formed on a front end and vertically reciprocates in a first direction from the other surface to the one surface of the housing (101) or a second direction from the one surface to the other surface of the housing (101) through the first nozzle portion (1041) and the second nozzle portion (1042); and
a second valve portion (1052) which is formed on a lower portion of the first valve portion (1051), and
wherein the first valve portion (1051) is formed to have a diameter smaller than a diameter of the second valve portion (1052).

6. The inhaler (10) of claim 5, wherein the first valve portion (1051) is formed to have the diameter smaller than the diameter of the first nozzle portion (1041).

7. The inhaler (10) of claim 5,
wherein the first valve portion (1051) does not come into contact with the first nozzle portion (1041) in the first state or the second state, and
wherein the second valve portion (1052) comes into contact with the sealing member (1043) in the first state and does not come into contact with the sealing member (1043) in the second state.

8. The inhaler (10) of claim 5,
wherein the first gap (G1) is formed between the first valve portion (1051) and the first nozzle portion (1041), and
wherein the second gap (G2) is formed between the first valve portion (1051) and the sealing member (1043).

9. The inhaler (10) of claim 1,
wherein the second gap (G2) allows movement of the inhalable composition discharged from the reservoir (103), and
wherein the first gap (G1) allows the movement of the inhalable composition to the mouthpiece (102) by controlling a particle size of the inhalable composition passing through the second gap (G2).

10. The inhaler (10) of claim 1, wherein the first gap (G1) is formed to 0.015 millimeters (mm) to 0.03 mm.

11. The inhaler (10) of claim 1, further comprising:
a piston (106) which has one surface coupled to the needle valve (105), and vertically reciprocates in a first direction from the other surface to the one surface of the housing (101) or a second direction from the one surface to the other surface of the housing (101); and
a spring (107) which is provided below the piston in a preloaded state,
wherein, when a suction force is not applied through the mouthpiece (102), the spring (107) pushes the piston (106) up in the first direction to maintain the first state, and
wherein, when a suction force is applied through the mouthpiece (102), the piston (106) overcomes preload of the spring (107) and moves in the second direction to switch the state into the second state.

12. The inhaler (10) of claim 11, further comprising:
a passage (108) which is formed inside the housing (101) and extends from one side of the mouthpiece (102) to a bottom portion of the piston (106),
wherein the passage (108) transfers the suction force applied to the mouthpiece (102) to the piston (106).

13. The inhaler (10) of claim 12, further comprising:
a negative pressure forming portion (109) which forms a space between the piston (106) and the passage (108),
wherein the negative pressure forming portion (109) induces the piston (106) to move in the second direction by generating a negative pressure by the suction force.

## Patentansprüche

1. Inhalationsvorrichtung (10), die Folgendes umfasst:
ein Gehäuse (101), das eine Fläche, die weitere Fläche, die der einen Fläche gegenüberliegt, und mehrere Seitenflächen, die die eine Fläche und die weitere Fläche verbinden, aufweist;
ein Mundstück (102), das auf der einen Fläche des Gehäuses (101) angeordnet ist;
einen Vorratsbehälter (103), der im Inneren des Gehäuses (101) angeordnet ist und eine inhalierbare Zusammensetzung speichert;
eine Düse (104), die sich vom Mundstück (102) zum Vorratsbehälter (103) erstreckt;
ein Nadelventil (105), das im Inneren der Düse (104) beweglich angeordnet ist und in einem ersten Zustand zum Verschließen der Düse (104) und einem zweiten Zustand zum Öffnen der Düse (104) arbeitet; und
ein Dichtungselement (1043), das an der Düse (104) fest vorgesehen ist,
wobei im ersten Zustand ein erster Zwischenraum (G1) zwischen dem Nadelventil (105) und der Düse (104) gebildet wird,
wobei im zweiten Zustand ein zweiter Zwischenraum (G2) zwischen dem Nadelventil (105) und dem Dichtungselement (1043) gebildet wird,
wobei das Dichtungselement (1043) als ein Quad-Ring vorgesehen ist, der aus einem elastischen Material gebildet ist, und
wobei eine Toleranz des Quad-Rings 0,05 mm ist.

2. Inhalationsvorrichtung (10) nach Anspruch 1,
wobei der erste Zustand als ein Zustand definiert ist, in dem das Nadelventil (105) mit dem Dichtungselement (1043) in Kontakt gelangt, und
wobei der zweite Zustand als ein Zustand definiert ist, in dem das Nadelventil (105) nicht mit dem Dichtungselement (1043) in Kontakt gelangt.

3. Inhalationsvorrichtung (10) nach Anspruch 1, wobei der erste Zwischenraum (G1) kleiner als der zweite Zwischenraum (G2) ist.

4. Inhalationsvorrichtung (10) nach Anspruch 1,
wobei die Düse (104) Folgendes umfasst:
einen ersten Düsenabschnitt (1041), der an das Mundstück (102) angrenzt; und
einen zweiten Düsenabschnitt (1042), der an den Vorratsbehälter (103) angrenzt,
wobei der erste Düsenabschnitt (1041) derart ausgebildet ist, dass er einen Durchmesser aufweist, der kleiner als ein Durchmesser des zweiten Düsenabschnitts (1042) ist.

5. Inhalationsvorrichtung (10) nach Anspruch 4,
wobei das Nadelventil (105) Folgendes umfasst:
einen ersten Ventilabschnitt (1051), der an einem vorderen Ende gebildet ist und sich in einer ersten Richtung von der weiteren Fläche zu der einen Fläche des Gehäuses (101) oder einer zweiten Richtung von der einen Fläche zu der weiteren Fläche des Gehäuses (101) durch den ersten Düsenabschnitt (1041) und den zweiten Düsenabschnitt (1042) vertikal hin und her bewegt; und
einen zweiten Ventilabschnitt (1052), der auf einem unteren Abschnitt des ersten Ventilabschnitts (1051) gebildet ist,
wobei der erste Ventilabschnitt (1051) derart ausgebildet ist, dass er einen Durchmesser aufweist, der kleiner als ein Durchmesser des zweiten Ventilabschnitts (1052) ist.

6. Inhalationsvorrichtung (10) nach Anspruch 5, wobei der erste Ventilabschnitt (1051) derart ausgebildet ist, dass er den Durchmesser aufweist, der kleiner als der Durchmesser des ersten Düsenabschnitts (1041) ist.

7. Inhalationsvorrichtung (10) nach Anspruch 5,
wobei der erste Ventilabschnitt (1051) im ersten Zustand oder im zweiten Zustand nicht mit dem ersten Düsenabschnitt (1041) in Kontakt gelangt, und
wobei der zweite Ventilabschnitt (1052) im ersten Zustand mit dem Dichtungselement (1043) in Kontakt gelangt und im zweiten Zustand nicht mit dem Dichtungselement (1043) in Kontakt gelangt.

8. Inhalationsvorrichtung (10) nach Anspruch 5,
wobei der erste Zwischenraum (G1) zwischen dem ersten Ventilabschnitt (1051) und dem ersten Düsenabschnitt (1041) gebildet wird, und
wobei der zweite Zwischenraum (G2) zwischen dem ersten Ventilabschnitt (1051) und dem Dichtungselement (1043) gebildet wird.

9. Inhalationsvorrichtung (10) nach Anspruch 1,
wobei der zweite Zwischenraum (G2) eine Bewegung der inhalierbaren Zusammensetzung, die aus dem Vorratsbehälter (103) ausgelassen wird, ermöglicht, und
wobei der erste Zwischenraum (G1) die Bewegung der inhalierbaren Zusammensetzung zum Mundstück (102) ermöglicht, indem eine Teilchengröße der inhalierbaren Zusammensetzung, die durch den zweiten Zwischenraum (G2) hindurchtritt, gesteuert wird.

10. Inhalationsvorrichtung (10) nach Anspruch 1, wobei der erste Zwischenraum (G1) im Bereich von 0,015 Millimetern (mm) bis zu 0,03 mm ausgebildet ist.

11. Inhalationsvorrichtung (10) nach Anspruch 1, die ferner Folgendes umfasst:
einen Kolben (106), der eine Fläche aufweist, die mit dem Nadelventil (105) gekoppelt ist, und sich in einer ersten Richtung von der weiteren Fläche zu der einen Fläche des Gehäuses (101) oder einer zweiten Richtung von der einen Fläche zu der weiteren Fläche des Gehäuses (101) vertikal hin und her bewegt; und
eine Feder (107), die in einem vorgespannten Zustand unter dem Kolben vorgesehen ist,
wobei dann, wenn durch das Mundstück (102) keine Saugkraft ausgeübt wird, die Feder (107) den Kolben in der ersten Richtung nach oben schiebt, derart, dass der erste Zustand aufrechterhalten wird, und
wobei dann, wenn durch das Mundstück (102) eine Saugkraft ausgeübt wird, der Kolben (106) die Vorspannung der Feder (107) überwindet und sich in der zweiten Richtung bewegt, derart, dass der Zustand in den zweiten Zustand umgeschaltet wird.

12. Inhalationsvorrichtung (10) nach Anspruch 11, die ferner Folgendes umfasst:
einen Kanal (108), der im Inneren des Gehäuses (101) gebildet ist und sich von einer Seite des Mundstücks (102) zu einem unteren Abschnitt des Kolbens (106) erstreckt,
wobei der Kanal (108) die Saugkraft, die auf das Mundstück (102) ausgeübt wird, zum Kolben (106) überträgt.

13. Inhalationsvorrichtung (10) nach Anspruch 12, die ferner Folgendes umfasst:
einen Unterdruckerzeugungsabschnitt (109), der einen Raum zwischen dem Kolben (106) und dem Kanal (108) bildet,
wobei der Unterdruckerzeugungsabschnitt (109) den Kolben (106) derart ansaugt, dass er sich in der zweiten Richtung bewegt, indem durch die Saugkraft ein Unterdruck erzeugt wird.

## Revendications

1. Inhalateur (10) comportant :
un boîtier (101) ayant une première surface, la seconde surface étant opposée à la première surface, et une pluralité de surfaces latérales reliant la première surface et la seconde surface ;
un embout buccal (102) qui est disposé sur la première surface du boîtier (101) ;
un réservoir (103) qui est disposé à l'intérieur du boîtier (101) et stocke une composition inhalable ;
une buse (104) qui s'étend à partir de l'embout buccal (102) jusqu'au réservoir (103) ;
une soupape à pointeau (105) qui est disposée de manière mobile à l'intérieur de la buse (104) et fonctionne dans un premier état destiné à fermer la buse (104) et dans un second état destiné à ouvrir la buse (104) ; et
un élément d'étanchéité (1043) qui est disposé de manière fixe sur la buse (104),
dans lequel, dans le premier état, un premier interstice (G1) est formé entre la soupape à pointeau (105) et la buse (104),
dans lequel, dans le second état, un second interstice (G2) est formé entre la soupape à pointeau (105) et l'élément d'étanchéité (1043),
dans lequel l'élément d'étanchéité (1043) est fourni sous la forme d'un joint à quatre lobes formé d'un matériau élastique, et
dans lequel une tolérance du oint à quatre lobes est de 0,05 mm.

2. Inhalateur (10) selon la revendication 1,
dans lequel le premier état est défini comme un état dans lequel la soupape à pointeau (105) vient en contact avec l'élément d'étanchéité (1043), et
dans lequel le second état est défini comme un état dans lequel la soupape à pointeau (105) ne vient pas en contact avec l'élément d'étanchéité (1043).

3. Inhalateur (10) selon la revendication 1, dans lequel le premier interstice (G1) est plus petit que le second interstice (G2).

4. Inhalateur (10) selon la revendication 1,
dans lequel la buse (104) comporte :
une première partie de buse (1041) adjacente à l'embout buccal (102) ; et
une seconde partie de buse (1042) adjacente au réservoir (103), et
dans lequel la première partie de buse (1041) est formée de manière à avoir un diamètre inférieur au diamètre de la seconde partie de buse (1042).

5. Inhalateur (10) selon la revendication 4,
dans lequel la soupape à pointeau (105) comporte :
une première partie de soupape (1051) qui est formée sur une extrémité avant et est animée d'un mouvement de va-et-vient vertical dans une première direction allant de la seconde surface à la première surface du boîtier (101) ou dans une seconde direction allant de la première surface à la seconde surface du boîtier (101) à travers la première partie de buse (1041) et la seconde partie de buse (1042) ; et
une seconde partie de soupape (1052) qui est formée sur une partie inférieure de la première partie de soupape (1051), et
dans lequel la première partie de soupape (1051) est formée de manière à avoir un diamètre inférieur à un diamètre de la seconde partie de soupape (1052).

6. Inhalateur (10) selon la revendication 5, dans lequel la première partie de valve (1051) est formée de manière à avoir le diamètre inférieur au diamètre de la première partie de buse (1041).

7. Inhalateur (10) selon la revendication 5,
dans lequel la première partie de soupape (1051) ne vient pas en contact avec la première partie de buse (1041) dans le premier état ou le second état, et
dans lequel la seconde partie de soupape (1052) vient en contact avec l'élément d'étanchéité (1043) dans le premier état et ne vient pas en contact avec l'élément d'étanchéité (1043) dans le second état.

8. Inhalateur (10) selon la revendication 5,
dans lequel le premier interstice (G1) est formé entre la première partie de soupape (1051) et la première partie de buse (1041), et
dans lequel le second interstice (G2) est formé entre la première partie de soupape (1051) et l'élément d'étanchéité (1043).

9. Inhalateur (10) selon la revendication 1,
dans lequel le second interstice (G2) permet un mouvement de la composition inhalable évacuée du réservoir (103), et
dans lequel le premier interstice (G1) permet le mouvement de la composition inhalable jusqu'à l'embout buccal (102) en commandant une taille de particule de la composition inhalable passant à travers le second interstice (G2).

10. Inhalateur (10) selon la revendication 1, dans lequel le premier interstice (G1) est formé de 0,015 millimètre (mm) à 0,03 mm.

11. Inhalateur (10) selon la revendication 1, comportant en outre :
un piston (106) qui a une surface couplée à la soupape à pointeau (105) et qui est animé d'un mouvement de va-et-vient vertical dans une première direction allant de la seconde surface à la première surface du boîtier (101) ou dans une seconde direction allant de la première surface à la seconde surface du boîtier (101) ; et
un ressort (107) qui est agencé sous le piston dans un état préchargé,
dans lequel, lorsqu'une force d'aspiration n'est pas appliquée à travers l'embout buccal (102), le ressort (107) pousse le piston (106) vers le haut dans la première direction pour maintenir le premier état, et
dans lequel, lorsqu'une force d'aspiration est appliquée à travers l'embout buccal (102), le piston (106) surmonte une précharge du ressort (107) et se déplace dans la seconde direction pour basculer l'état dans le second état.

12. Inhalateur (10) selon la revendication 11, comportant en outre :
un passage (108) qui est formé à l'intérieur du boîtier (101) et s'étend à partir d'un côté de l'embout buccal (102) jusqu'à une partie inférieure du piston (106),
dans lequel le passage (108) transfère au piston (106) la force d'aspiration appliquée à l'embout buccal (102).

13. Inhalateur (10) selon la revendication 12, comportant en outre :
une partie de formation de pression négative (109) qui forme un espace entre le piston (106) et le passage (108),
dans lequel la partie de formation de pression négative (109) amène le piston (106) à se déplacer dans la seconde direction en générant une pression négative par la force d'aspiration.
